# EUROPEAN PATENT SPECIFICATION

(11) **EP 1 781 304 B1**
(45) Date of publication and mention of the grant of the patent: **09.09.2009**
(21) Application number: 05752692.3
(22) Date of filing: 23.06.2005
(51) Int. Cl.: A61K 31/7008, A61P 19/02

(54) **USE OF ORGANIC GLUCOSAMINE SALTS**
VERWENDUNG VON ORGANISCHEN GLUCOSAMINSALZEN
UTILISATION DE SELS ORGANIQUES DE GLUCOSAMINE

(30) Priority: 06.08.2004 ES 200401972
(43) Date of publication of application: 09.05.2007
(73) Proprietor: BIOIBERICA, S.A., 08029 Barcelona (ES)
(72) Inventor: ESCAICH FERRER, Josep, Bioiberica, S.A., E-08029 Barcelona (ES); RUHI ROURA, Ramon, E-08025 Barcelona (ES); TORRENT GIBERT, Ana, Maria, E-17452 Massanes (ES)
(74) Representative: Sugrañes Patentes y Marcas
(86) International application number: PCT/EP2005/006804
(87) International publication number: WO 2006/012951

(56) References cited:
- EP-A- 0 444 000
- US-A1- 2004 092 482
- DODGE G R ET AL: "Glucosamine sulfate modulates the levels of aggrecan and matrix metalloproteinase-3 synthesized by cultured human osteoarthritis articular chondrocytes" OSTEOARTHRITIS AND CARTILAGE 01 JUN 2003 UNITED KINGDOM, vol. 11, no. 6, 1 June 2003 (2003-06-01), pages 424-432, XP002344589 ISSN: 1063-4584

## Description

### Technical field of the invention

This invention relates to the use of an organic glucosamine salt selected from the group consisting of glucosamine malate and glucosamine hydrogen malate for the treatment or prophylaxis of osteoarthritis, of the inflammation associated with osteoarthritis, and the pain associated with osteoarthritis. Likewise, this invention relates to the use of an organic glucosamine salt selected from the group consisting of glucosamine malate and glucosamine hydrogen malate for the preparation of a nutritional supplement acting as a chondroprotector, as a cartilage nutrient, as a joint protector, to prevent water deficit in the tissues that form the joints, to improve the joints' functional capacity, elasticity, and flexibility, and for the prophylaxis and reversion of the physical overexertion syndrome in athletes and the undesirable effects associated therewith.

### Description of the prior art

Osteoarthritis, also known as arthrosis, is a degenerative joint disease which affects most people over 65 years of age, characterised by a gradual degradation of the cartilaginous tissue, together with the presence of inflammation and pain. Synovial inflammation normally appears later, when the disease is at an advanced stage and is different in nature from the inflammation observed in rheumatoid arthritis, and generally is only a minority component of arthrosic pathology. Osteoarthritis may be defined as the degeneration of the hyaline articular cartilage. A secondary effect thereto is affectation of the synovial membrane and the subchondral bone, as well as the formation of new bone on the margins of the joint surfaces. The etiology of osteoarthritis is unknown and its evolution is slow. Depending on its etiology, osteoarthritis may be divided into two groups. The first would be primary osteoarthritis, wherein the cartilage begins to degenerate and become injured without a known cause having been determined, and secondary osteoarthritis, which is related to the presence of mechanical alterations or problems in the joint, with wounds, infections, metabolic disorders, or other dysfunctions. However, a series of risk factors for the appearance of the disease have been described, such as: ageing, inheritance, obesity, overloading disorders, physical overexertion in athletes, injuries or traumas, bone mineral density, among others.

The cartilage allows bones to move, slipping over one another. It also absorbs the stress produced by physical movement. In osteoarthritis, the surface of the cartilage breaks and wears out, causing bones to move against one another, which leads to friction, pain, swelling, and loss of joint movement. As time goes on, the joint may deform.

Under normal conditions, cartilage renewal is a very slow process, which consists of a constant synthesis (anabolism) and degradation (catabolism) of the extracellular matrix components. The chondrocyte is the cell responsible for this metabolism, which must be coordinated.

Under pathological conditions, this process is altered, because cartilage renewal is accelerated, which leads to a precocious repair of the cartilaginous tissue caused by an imbalance between the chondrocyte's anabolic and catabolic programmes, which entails degradation of the cartilage. The repair reaction is the result of a hyperproliferation of chondrocytes, together with an increase in the synthesis of the cartilage's extracellular matrix components by these cells (D. Hamermam et al., N. Engl. J. Med., 320, 1322-1330 (1989)). Consequently, there exists a balance between synthesis and degradation of the cartilage which controls this homeostatic reaction and which depends on systemic hormones and growth factors whose secretions decrease with age. Cartilage degradation is regulated by enzymes and free radicals produced by adjacent tissues, but also by the chondrocyte itself.

We will highlight the following current pharmacological treatments for osteoarthritis:
Symptomatic-action substances which have a rapid action, such as analgesics, non-steroidal anti-inflammatory drugs (NSAIDs), corticoids, and cyclooxygenase 2 inhibitors (COX-2). The use of some of them entails a high risk of potentially severe secondary effects, such as gastrointestinal problems in the case of NSAIDs.
Symptomatic-action substances which act in a slower manner, known as SYSADOA (Symptomatic Slow Acting Drug for Osteoarthritis) (M.G. Lequesne, Rev. Rhum. (Eng./Ed.), 61, 69-73 (1994)), include hyaluronic acid, chondroitin sulphate, glucosamine hydrochloride, and the so-called glucosamine sulphate. This group is
characterised by having as additional advantages a greater safety by comparison with NSAIDs and a more prolonged action, of even several months after the suppression of treatment.

Glucosamine, which is an aminomonosaccharide, is an intermediate substrate used by the joint cartilage in the synthesis of glycosaminoglycans and proteoglycans. Glucosamine is present as a natural compound in almost all human tissues.

Various research groups are still studying the effects of glucosamine hydrochloride on osteoarthritis. H. Nakamura et al. (Clin. Exp. Rheumatol. 22 (3), 293-9, (2004)) describe the effects of glucosamine hydrochloride in the production of prostaglandin E2, nitric oxide, and metalloproteases by chondrocytes and synoviocytes in osteoarthritis. These researchers conclude that glucosamine hydrochloride modulates the metabolism of chondrocytes and synoviocytes.

Some researchers looked for an alternative to the use of glucosamine hydrochloride in the treatment of osteoarthritis, by attempting to stabilise glucosamine sulphate, either by adding other components (EP 444,000 B1) or by forming the so-called mixed salts (WO 99/61455).

JP2002-187846 discloses inorganic and organic salts of glucosamine (chloride, sulfate, lactate and ascorbate) as anti-arthritic agents.

From all of the above, one may conclude that providing other glucosamine salts as an alternative to the glucosamine hydrochloride salt in the treatment of osteoarthritis and other conditions associated with arthrosic pathology, as well as for the preparation of nutritional supplements to act on the cartilage, the joints, and the undesirable effects produced by physical overexertion in athletes is still a therapeutic and nutritional problem.

Until now no description has been found of the use of glucosamine malate or glucosamine hydrogen malate in the treatment or prophylaxis of osteoarthritis, the inflammation associated with osteoarthritis or the pain associated with osteoarthritis.

We also have not found any description of the use of glucosamine malate or glucosamine hydrogen malate for the preparation of a nutritional supplement to act as a chondroprotector, as a cartilage nutrient, or to act as a joint protector or in the prophylaxis and reversion of water deficit in the tissues which form the joint or in order to enhance the joints' functional capacity, elasticity, and flexibility, or in the prophylaxis and reversion of the physical overexertion syndrome in athletes and the undesirable effects associated therewith, particularly bone, joint, muscle, and cartilage injuries.

### Disclosure of the invention

The present invention relates to the use of an organic glucosamine salt selected from the group consisting of glucosamine malate and glucosamine hydrogen malate, for the preparation of a medicament for the treatment, prevention, or prophylaxis of osteoarthritis in a mammal.

According to another characteristic, said treatment results in the treatment of inflammation associated with osteoarthritis in said mammal.

According to another characteristic, said treatment results in the treatment of pain associated with osteoarthritis in said mammal.

In a preferred embodiment, the organic glucosamine salt is glucosamine malate or glucosamine hydrogen malate.

In an equally preferred embodiment, the medicament is adapted for oral administration.

According to another characteristic, the medicament is in the form of a liquid, solid, in emulsion, in suspension or in a gel nutritional supplement.

In an equally preferred embodiment, the medicament is adapted for intra-articular administration.

According to another characteristic, the medicament is a chondroprotector in the form of a nutritional supplement.

When we speak herein about glucosamine malate, this relates to diglucosamine malate, i.e. the salt that is formed when the two carboxyl groups react.

There are various known procedures for the preparation of glucosamine salts. Some of them consist of previously obtaining the glucosamine base from glucosamine hydrochloride, and subsequently adding the corresponding acid, depending on the salt one wishes to obtain. In general, in order to obtain glucosamine base, glucosamine hydrochloride is treated with triethylamine (L. Rovati, CH 525.861), or with sodium methoxide (L. Rovati, US 3.683.076), or also by means of anionic exchange resins. The salts may also be directly obtained from glucosamine hydrochloride and using an anionic exchange resin previously conditioned with the acid containing the anion of the salt one wishes to produce, or else an acid-metal salt, or they may also be directly produced from glucosamine hydrochloride and an acid-metal salt. If the organic acid contains more than one carboxyl group, by varying the starting quantity of glucosamine or glucosamine hydrochloride, we will obtain the desired salt.

For use in the treatment or prophylaxis of osteoarthritis, in the treatment of inflammation associated with osteoarthritis, and in the treatment of pain associated with osteoarthritis, the salts in the invention are formulated in appropriate pharmaceutical compositions, using conventional techniques and excipients or vehicles, such as the ones described in Remington's Pharmaceutical Sciences Handbook, Mack Pub. Co., N.Y., USA.

The pharmaceutical compositions in the invention may be administered to the patient in required doses. Administration of the compositions may be performed by various means, for example, oral, intravenous, intraperitoneal, intra-articular, subcutaneous, intramuscular, topical, sublingual, intradermal, or intranasal. The pharmaceutical compositions in the invention include a therapeutically effective quantity of the salt in this invention, with said quantity being dependent on many factors, such as, for instance, the patient's physical condition, age, sex, specific compound, means of administration, and other factors well-known in technology. Furthermore, it is understood that said dosage of the active compound may be administered in single- or multiple-dose units in order to provide the desired therapeutic effects. If so desired, other therapeutic agents may be used jointly with the ones provided by this invention.

In general, the pharmaceutical preparations in the invention will be in solid or liquid form, or in the form of a gel. The solid-form pharmaceutical preparations which may be prepared in accordance with the present invention include powders, minigranules (pellets), tablets, dispersable granules, capsules, suppositories, and other solid galenical forms. The liquid-form preparations include solutions, suspensions, emulsions, and microspheres. Preparations in solid form which, immediately before being used, may be converted to liquid preparations for oral, parenteral, or intra-articular administration, are also contemplated. Said liquid forms include solutions, suspensions, and emulsions.

In order to prepare the nutritional supplements to be used in accordance with the invention, the salts in the invention are formulated with appropriate components and excipients used in nutrition. The nutritional supplements may be, for instance, in solid or liquid form, in the form of emulsions or suspensions, or as a gel.

### Brief description of the figure

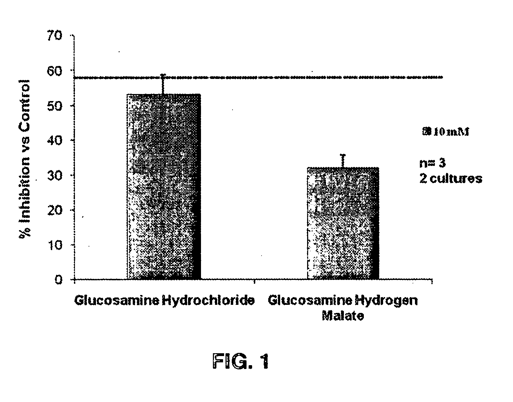

- Figure 1: represents the results of the inhibition of aggrecanolysis induced by IL-1a, both for glucosamine hydrochloride and for glucosamine hydrogen malate with respect to the control. The control represents
the culture to which only IL-1 α has been added.

### Detailed description of the invention

### Chemical Examples

### Example 1: Preparation of glucosamine hydrogen malate

Under constant stirring, 1 kg of finely ground glucosamine hydrochloride (preferably 100 % < 200 microns) was added to 0.77 L of distilled water contained in a three neck flask.

The stirring was continued and, while maintaining the temperature at 10-20°C, 0.562 kg of triethylamine was added for 20-30 minutes. Subsequently, 1.64 L of methanol were added and the stirring was continued for 30 minutes. The product was filtered, and the precipitate was re-suspended in 1.3 L of methanol and was once again stirred for 5 minutes. Subsequently, it was filtered once again. It was then washed with methanol three more times.

Once the product was filtered, it was dried in a vacuum stove at ambient temperature.

737 g of glucosamine base were produced, wherein it was verified that the content of residual chlorides was less than 0.5 %.

In order to obtain glucosamine hydrogen malate, the glucosamine base was introduced in a precipitation vessel and 2,948 mL of de-ionised water were added in order to prepare a 25% (w/v) solution. It was stirred until complete dissolution was achieved and, subsequently, 551.6 g of malic acid were slowly added, at a controlled temperature between 2-4 °C (approximately 1 hour).

Once the reaction was finished, in order to obtain the solid product, it was subject to a lyophilisation process; 1,280 g of a white solid were obtained, with a melting point of 132 °C with decomposition.
Richness: 95.4 %
Chloride content: 0.3 %
Formation of the salt was confirmed by the absence of chlorides.
This procedure of preparation may also be applied to synthesize other glucosamine salts like for example:

### Example 2: Preparation of glucosamine glucuronate

The procedure described in Example 1 was followed, starting from glucosamine base and glucuronic acid.
Melting point: 118 °C with decomposition.
Richness: 94.0 %
Chloride content: 0.1 %
Formation of the salt was confirmed by H-NMR and by the absence of chlorides.

### Example 3: Preparation of glucosamine dihydrogen citrate

The procedure described in Example 1 was followed, starting from glucosamine base and citric acid.
Melting point: 142 ºC with decomposition.
Richness: 94.6 %
Chloride content: 0.24 %
Formation of the salt was confirmed by H-NMR and by the absence of chlorides.
The ¹H-RMN shows a 0.12 ppm displacement of the signal corresponding to the - CH₂- whose -COO- is forming the salt with glucosamine.

### Example 4: Preparation of glucosamine ascorbate

The procedure described in Example 1 was followed, starting from glucosamine base and ascorbic acid.
Melting point: 135 °C with decomposition.
Richness: 93.8 %
Chlorides: 0.4 %
Formation of the salt was confirmed by ¹H-NMR and by the absence of chlorides.

### Pharmacological examples

### Example 1: Determination of glucosamine hydrogen malate's capacity to inhibit aggrecan degradation induced with IL-1α

The study of the inhibition of aggrecan degradation was performed on a chondrocyte culture from a rat chondrosarcoma cell line. This cell line, called LTC, deposits an extracellular matrix similar to the cartilage's, expresses all the components of the aggrecanase system, generates the fragmentation of aggrecan by said enzymes, and responds to known aggrecanolysis inhibitors such as glucosamine hydrochloride.

This procedure may also be applied to evaluate the effectiveness of glucosamine malate or any other glucosamine salts.

### Materials and methods

The assays were performed on chondrocyte cultures from a rat chondrosarcoma cell line called LTC.

The LTC cells were kept in a monolayer culture in a Gibco DMEM medium (Dulbecco's Modification of Eagle's Medium) (containing 4.5 g/L of glucose) supplemented with sodium bicarbonate (3.7 g/L), glutamine (2mM), ascorbic acid (50 mg/L), gentamicin (50 mg/L), and bovine fetal serum Hyalone (10%), at pH 7.4.

The confluent LTC cell cultures were trypsinised and 40,000 cells per 0.5 mL of culture were seeded in 48-well plates. They were maintained for 5 days, during which time they deposited 15-30 µg of glycosaminoglycans (GAG) in each well.

The medium was separated, the cell membranes were washed by adding 5 x 1 mL of a catabolic medium {DMEM + 4.5 g/L glucose supplemented with sodium bicarbonate (3.7 g/L), glutamine (2 mM), and 10 ng/mL of IL-1α (interleukin-1α) at pH 7.4}. Subsequently, the cultures were kept in 200 µL of this medium, supplemented or not with glucosamine hydrogen malate, for 4 days, without changing the medium, at 37 °C.

In order to verify the effect of glucosamine hydrogen malate on aggrecan degradation, a Western Blot was performed.

For the Western Blot, 20 µL of 50 mM tris-acetate at pH 7.3 were added to each well. The cultures (medium + cell membrane) were deglycosylated with 50 mU of chondroitinase ABC at 37 °C for 4 hours. Subsequently, the samples in each well were recovered and centrifuged at 1,460 g for 10 minutes. 25 µL-fractions of the supernatant were separated by electrophoresis (gel gradient 8-12 % SDS PAGE) and analysed by means of a Western Blot using anti-G1 antibody. The antiserum was used at a 1:5,000 dilution and the peroxidase-conjugated goat anti-(rabbit IgG) was detected by means of the Amersham ECL Kit. The exposure time ranged between 5 seconds and 5 minutes in order to obtain quantifiable images. The Western Blots were scanned and the bands were quantified by means of the SCION image analysis software. In relation to the described procedure, see: J.D. Sandy et al., Biochem. J. 335, 59-66 (1998).

### Results

Figure 1 represents the results of the inhibition of aggrecanolysis induced by IL-1α, both for glucosamine hydrochloride and for glucosamine hydrogen malate with respect to the control. The control represents the culture to which only IL-1α has been added.

The results of inhibition vs. the control are the following:
10mM glucosamine hydrochloride: 53 % ± 5.8
10mM glucosamine hydrogen malate: 32 % ± 3.7

As can be seen in Figure 1, 10 mM glucosamine hydrogen malate is significantly more effective than glucosamine hydrochloride.

## Claims

1. Use of an organic glucosamine salt selected from the group consisting of glucosamine malate and glucosamine hydrogen malate, for the preparation of a medicament for the treatment or prophylaxis of osteoarthritis in a mammal.

2. Use according to claim 1, wherein the treatment results in the treatment of inflammation associated with osteoarthritis in said mammal.

3. Use according to claim 1, wherein the treatment results in the treatment of pain associated with osteoarthritis in said mammal.

4. Use according to any one of claims 1 to 3, wherein the organic glucosamine salt is glucosamine malate.

5. Use according to any one of claims 1 to 3, wherein the organic glucosamine salt is glucosamine hydrogen malate.

6. Use according to any one of claims 1 to 5, wherein the medicament is adapted for oral administration.

7. Use according to any one of claims 1 to 5, wherein the medicament is adapted for intra-articular administration.

8. Use according to any one of claims 1 to 6, wherein the medicament is in the form of a liquid, solid, in emulsion, in suspension or in a gel nutritional supplement.

9. Use according to any one of claims 1 to 6, wherein the medicament is a chondroprotector in the form of a nutritional supplement.

## Patentansprüche

1. Verwendung eines organischen Glucosaminsalzes ausgewählt aus der Gruppe bestehend aus Glucosamin-Malat und Glucosamin-Hydrogenmalat, zur Aufbereitung eines Medikaments zur Behandlung oder Prophylaxe von Arthrose bei einem Säugetier.

2. Verwendung nach Anspruch 1, bei der die Behandlung die Behandlung einer mit Arthrose in Verbindung stehender Entzündung in dem genannten Säugetier zur Folge hat.

3. Verwendung nach Anspruch 1, bei der die Behandlung die Behandlung von mit Arthrose in Verbindung stehenden Schmerzen zur Folge hat.

4. Verwendung nach einem der Ansprüche 1 bis 3, bei der das organische Glucosaminsalz Glucosamin-Malat ist.

5. Verwendung nach einem der Ansprüche 1 bis 3, bei der das organische Glucosaminsalz Glucosamin-Hydrogenmalat ist.

6. Verwendung nach einem der Ansprüche 1 bis 5, bei der das Medikament zur oralen Verabreichung angepasst ist.

7. Verwendung nach einem der Ansprüche 1 bis 5, bei der das Medikament zur intraartikulären Verabreichung angepasst ist.

8. Verwendung nach einem der Ansprüche 1 bis 6, bei der das Medikament als Nahrungsergänzungsmittels in flüssiger, fester, Emulsions-, Suspensions- oder Gelform vorliegt.

9. Verwendung nach einem der Ansprüche 1 bis 6, bei der das Medikament ein Chondroprotector in Form eines Nahrungsergänzungsmittels ist.

## Revendications

1. Utilisation d'un sel organique de glucosamine choisi dans le groupe constitué par le malate de glucosamine et l'hydrogénomalate de glucosamine pour la préparation d'un médicament pour le traitement ou la prophylaxie d'arthrose chez un mammifère.

2. Utilisation selon la revendication 1, dans laquelle le traitement donne comme résultat le traitement d'inflammation associé à l'arthrose chez ledit mammifère.

3. Utilisation selon la revendication 1, dans laquelle le traitement donne comme résultat le traitement de douleur associé à l'arthrose chez ledit mammifère.

4. Utilisation selon l'une quelconque des revendications 1 à 3, dans laquelle le sel organique de glucosamine est du malate de glucosamine.

5. Utilisation selon l'une quelconque des revendications 1 à 3, dans laquelle le sel organique de glucosamine est de l'hydrogénomalate de glucosamine.

6. Utilisation selon l'une quelconque des revendications 1 à 5, dans laquelle le médicament est adapté pour son administration orale.

7. Utilisation selon l'une quelconque des revendications 1 à 5, dans laquelle le médicament est adapté pour son administration intra-articulaire.

8. Utilisation selon l'une quelconque des revendications 1 à 6, dans laquelle le médicament est sous forme d'un liquide, solide, en émulsion, en suspension ou dans un supplément nutritionnel en gel.

9. Utilisation selon l'une quelconque des revendications 1 à 6, dans laquelle le médicament est un chondroprotecteur sous forme d'un supplément nutritionnel.
